Europäisches Patentamt

European Patent Office

Office européen des brevets

(19)

(11) Numéro de publication : **0 087 993**
**B2**

(12) NOUVEAU FASCICULE DE BREVET EUROPÉEN

(45) Date de publication du nouveau fascicule du brevet :
03.01.90

(51) Int. Cl.⁵ : **A 61 K 9/50**, A 61 K 7/00

(21) Numéro de dépôt : 83400281.8

(22) Date de dépôt : 09.02.83

(54) **Mélange pulvérulent de constituants lipidiques et de constituants hydrophobes, procédé pour le préparer, phases lamellaires lipidiques hydratées et procédé de fabrication, compositions pharmaceutiques ou cosmetiques comportant des phases lamellaires lipidiques hydratées.**

(30) Priorité : 17.02.82 FR 8202620

(43) Date de publication de la demande :
07.09.83 Bulletin 83/36

(45) Mention de la délivrance du brevet :
02.07.86 Bulletin 86/27

(45) Mention de la décision concernant l'opposition :
03.01.90 Bulletin 90/01

(84) Etats contractants désignés :
AT BE CH DE FR GB IT LI LU NL SE

(56) Documents cités :
EP–B– 0 043 327
DE–A– 2 451 568
FR–A– 2 315 991
FR–A– 2 399 241
FR–A– 2 416 008
FR–A– 2 446 635
GB–A– 1 575 344
US–A– 3 499 962
HAGERS HANDBUCH DER PHARMAZEUTISCHEN PRAXIS, 4ième édition, point VIIB, 1971, Springer-Verlag, Berlin, DE
K. Masters, I. Vestergaard, Process Biochemistry, Jan./Feb. 1975, "Aseptic and Closed Cycle Spray Drying in Pharmaceutical/Biochemicals Manufacture"

(73) Titulaire : PARFUMS CHRISTIAN DIOR
30 avenue Hoche
F-75008 Paris (FR)

(72) Inventeur : Redziniak, Gérard, Mr.
40 rue Jacques Duclos
F-78500 Sartrouville (FR)
Inventeur : Meybeck, Alain, Mr.
"Les Poissons" 20 ter rue de Bezons
F-92400 Courbevoie (FR)

(74) Mandataire : Portal, Gérard et al
Cabinet Z. Weinstein 20, Avenue de Friedland
F-75008 Paris (FR)

EP 0 087 993 B2

## Description

La présente invention concerne généralement des phases lamellaires lipidiques hydratées et leur procédé de fabrication, ainsi que des compositions notamment à usage pharmaceutique, cosmétique ou analogues comprenant ces phases lamellaires lipidiques.

La présente invention a plus spécialement pour objet un procédé de préparation de mélanges pulvérulents de constituants lipidiques amphiphiles et de constituants hydrophobes, ledit mélange étant utilisé notamment pour la production de phases lamellaires lipidiques hydratées.

Les phases lamellaires lipidiques hydratées ont été abondamment décrites dans les publications scientifiques notamment par Perron R., (Revue Française du Corps Gras 1980, 27 (4) 173-183). Leur formation résulte d'une propriété caractéristique de certains lipides amphiphiles en présence d'un milieu aqueux. Les molécules des lipides amphiphiles sont constituées d'une partie hydrophile polaire ou non polaire et d'une partie hydrophobe. D'après M. M. Rieger (Cosmetics and Toiletries 1981, 96 35-38) une des conditions pour que le lipide amphiphile soit susceptible de former des phases lamellaires et pas seulement des agrégats micellaires est que l'encombrement de sa partie hydrophobe doit être au moins aussi important que celui de sa partie hydrophile.

Ces lipides sont susceptibles de former des couches lipidiques bimoléculaires (lamelles ou bicouches), séparées les unes des autres par une quantité plus ou moins importante d'eau ou de solution aqueuse qui peut être réduite à un simple film interstitiel. Les molécules desdits lipides formant des couches lipidiques sont placées les unes à côté des autres et orientées de manière à ce que leur partie hydrophobe soit située à l'intérieur de ladite couche. On appelle l'ensemble de ces couches lipidiques et du milieu aqueux interstitiel, une phase lamellaire lipidique hydratée.

Le liposome est une configuration particulière d'une phase lamellaire se présentant sous la forme d'une sphérule lipidique constituée d'une ou plusieurs bicouches concentriques alternant avec des compartiments aqueux. Les liposomes sont dispersés dans un milieu aqueux, et constituent une phase lamellaire très hydratée. Les conditions suivant lesquelles des lipides amphiphiles non polaires peuvent former des liposomes, ou « niosomes » ont été exposées par G. Vanlerberghe et al., (Colloques Nationaux du CNRS n° 938, Bordeaux 1978, Ed. CNRS 1979, p. 303-311).

On sait également que les liposomes peuvent être obtenus par dispersion dans un milieu aqueux d'une phase lamellaire, suivant divers procédés, notamment au moyen d'ultra-sons.

Les liposomes ont fait l'objet d'un grand nombre de publications, telles que par exemple les articles déjà cités et les articles suivants : Sessa G. et al., J. Lipid Res. 1968, 9, 310-318 ; Bangham A. D. et al., Meth. Membr. Biol. 1974, 1-68 ; D. A. Tyrrell et al., Biochim. Biophys. Acta 1976, 457, 259-302 ; Strianse S. J., Cosmetics & Toiletries 1978, 93, 37-41 ; Puisieux F., Labo-Pharma 1978, 281, 899-904 ; Nicolau C. et al., La Recherche 1981, 123, 748-749.

L'intérêt industriel des liposomes ou des phases lamellaires hydratées, réside dans le fait que des substances possédant des propriétés intéressantes peuvent être incorporées, soit dans les bicouches lipidiques pour les substances partiellement ou totalement hydrophobes, soit en solution dans le milieu aqueux situé entre deux bicouches ou encapsulées dans des liposomes pour les substances hydrosolubles.

Les applications de ces produits sont très nombreuses en particulier en alimentation, cosmétique ou pharmacie.

Des substances très variées peuvent être incorporées aux phases lamellaires ou aux liposomes, notamment dans un but de protection des substances fragiles contre les agents extérieurs, ou d'amélioration de la pénétration de certaines substances dans les tissus biologiques. Il est également possible de modifier les propriétés physico-chimiques des phases lamellaires ou des liposomes eux-mêmes par incorporation aux bicouches lipidiques de certains composés comme les stérols, par exemple le cholestérol (Vanderberghe G. et al., cité ci-dessus ; Grégoriadis G., Biochem J. 19, 196, 591) pour notamment contrôler la perméabilité et la stabilité des bicouches lipidiques.

L'addition des composés hydrophobes comme les stérols augmente le rapport groupement hydrophobe/groupement hydrophile dans la phase lamellaire, ce qui est en accord avec la condition de stabilité des phases lamellaires exposée par M. M. Rieger (cité ci-dessus). Ainsi, l'addition de tels composés hydrophobes rend possible la formation de phases lamellaires avec des lipides amphiphiles qui ne pourraient en former s'ils étaient employés seuls. Il est également possible d'incorporer aux phases lamellaires des composés amphiphiles dont la partie hydrophile sera chargée positivement ou négativement (demandes de brevets françaises no 78-13632 (FR-A-2 390159) et 78-22984 (FR-A-2 399 241), par exemple, le phosphate de dicétyle attribuant aux liposomes une charge négative, tandis que la stéarylamine apporte une charge positive.

Un certain nombre de procédés de production de telles phases lamellaires, et de liposomes sont déjà connus. Un des procédés le plus couramment utilisé consiste dans un premier temps à dissoudre le ou les lipides amphiphiles et éventuellement la ou les substances hydrophobes dans un solvant volatil approprié. La solution obtenue est placée dans le ballon d'un évaporateur rotatif. Après évaporation du solvant on obtient un film adhérent à la paroi du ballon. On introduit alors dans ce dernier de l'eau ou éventuellement la solution aqueuse à encapsuler et l'on soumet le tout à une vigoureuse agitation. On obtient alors une suspension de liposomes qui peut être, éventuellement, homogénéisée par ultra-sons. Un des inconvénients de ce procédé réside dans l'obtention du film qui ne peut être obtenu que très difficilement à une échelle industrielle.

Selon un autre procédé décrit notamment dans la demande française n° 78-22984 (FR-A-2 399241) la solution aqueuse à encapsuler est dispersée dans un solvant insoluble ou peu soluble dans l'eau en présence d'un lipide ou d'un surfactif amphiphile. Il se forme alors des microgouttes de ladite solution aqueuse que l'on émulsifie ensuite dans un milieu aqueux en présence d'un excès d'un lipide ou surfactif qui peut être identique ou différent de celui utilisé précédemment. Le solvant insoluble est éliminé avant ou après l'émulsification, par exemple par insuflation d'air à la surface du liquide. Ce procédé nécessite une élimination totale du solvant utilisé ce qui présente de nombreuses difficultés, notamment à une échelle industrielle. De plus, il ne permet pas la production de phases lamellaires peu hydratées.

Il est également possible de préparer des liposomes selon le brevet Suisse n° 623.236 en mettant en contact, d'une part, au moins un lipide amphiphile liquide dispersible dans l'eau, dont la partie hydrophile est ionique, et d'autre part, la phase aqueuse à encapsuler. Le mélange est alors soumis à une agitation vigoureuse pour obtenir une phase lamellaire qui peut être par la suite dispersée dans l'eau ou une solution aqueuse. Dans ce procédé, pour incorporer des composés hydrophobes, il est nécessaire d'employer des composés tensio-actifs à propriété émulsifiante d'autant plus forte que le caractère hydrophobe du composé à incorporer est plus marqué. Ces tensio-actifs sont très souvent incompatibles avec de nombreuses applications des liposomes par exemple pharmaceutiques ou cosmétiques.

Selon un autre procédé décrit dans la demande française n° 78-13632 (FR-A-2 390 159) on prépare une solution contenant au moins un lipide amphiphile, au moins un composé biologiquement actif ou éventuellement au moins un adjuvant. On lyophilise cette solution pour éliminer le solvant et le mélange des constituants est obtenu sous forme d'une poudre qui peut être conservée dans des récipients scellés jusqu'à son utilisation pour former notamment des liposomes. Cette dernière étape consiste à disperser ladite poudre dans un milieu aqueux convenable. Ce procédé présente des inconvénients liés notamment aux techniques classiques de lyophilisation qui sont relativement complexes et onéreuses et de plus qui ont un débit de production relativement limité.

Les différents procédés connus décrits ci-dessus présentent donc de nombreux inconvénients ne permettant pas notamment une production industrielle aisée ou peu coûteuse de phases lamellaires. Ces inconvénients sont principalement dus aux difficultés pour obtenir un mélange intime des constituants de base formant les phases lamellaires, en d'autres termes, les lipides amphiphiles et éventuellement les substances hydrophobes. Pour faciliter la dispersion des constituants de base des phases lamellaires dans un milieu aqueux, il est souvent nécessaire d'utiliser des produits additionnels à propriété tensio-active. Comme ces produits ont souvent des effets indésirables, par exemple vis-à-vis de l'organisme, il est nécessaire de les éliminer complètement, ce qui n'est pas toujours possible même par dialyse. On entend par l'expression « constituants de base », les composés utilisés pour former les couches lipidiques de la phase lamellaire lipidique, à savoir au moins un lipide amphiphile ou mélange de un ou plusieurs lipides amphiphiles et éventuellement d'un ou plusieurs composés partiellement ou totalement hydrophobes.

La présente invention a pour but de remédier à tous ces inconvénients en proposant un nouveau procédé de préparation de phases lamellaires lipidiques hydratées, et plus particulièrement un nouveau procédé de préparation d'un mélange pulvérulent de constituants lipidiques amphiphiles avec éventuellement des constituants hydrophobes ou partiellement hydrophobes. Ce procédé est d'une grande simplicité et permet de produire en continu de grandes quantités de mélanges et donc de phases lamellaires lipidiques hydratées ou de liposomes, sans pour cela nécessiter notamment l'utilisation de produits additionnels à propriété tensio-active.

A cet effet, la présente invention a pour objet un procédé de préparation d'un mélange pulvérulent apte à la production de phases lamellaires lipidiques hydratées, telles que par exemple des liposomes ledit mélange comprenant au moins un constituant lipidique amphiphile et, éventuellement au moins un constituant à caractère hydrophobe, caractérisé en ce qu'il comprend la mise en solution du ou desdits constituants lipidiques amphiphiles, et éventuellement du ou desdits constituants à caractère hydrophobe, dans un solvant non aqueux et l'atomisation de la solution ainsi obtenue dans un courant de fluide gazeux porté à une température dite température d'atomisation supérieure à la température d'ébullition du solvant précité pour produire ledit mélange pulvérulent, lequel solvant est choisi en fonction de son point d'ébullition qui ne doit pas être trop élevé pour éviter l'utilisation d'une température d'atomisation risquant de dégrader le ou lesdits constituants.

Ainsi, il est possible d'obtenir une poudre extrêmement fine qui peut être très facilement dispersée dans un milieu aqueux par des méthodes connues de dispersion, pour donner naissance à une phase lamellaire plus ou moins hydratée suivant le rapport de quantité de produit dispersé/quantité du milieu aqueux utilisé.

De plus, ce procédé permet d'obtenir un produit en poudre qui peut facilement être stocké en vue de son utilisation ultérieure, et donc ainsi de produire la phase lamellaire lipidique hydratée par dispersion de la poudre dans un milieu aqueux convenable, contenant éventuellement un composé actif à encapsuler immédiatement avant son utilisation par exemple comme médicament, ou produit traitant en dermatologie, cosmétique ou analogues, et plus particulièrement quand le composé à propriété active intéressante est relativement instable et ne peut être préparé qu'immédiatement avant son utilisation.

Le procédé de l'invention consiste donc à dissoudre dans un solvant ou un mélange de solvants appropriés les constituants de base, en d'autres termes au moins un lipide amphiphile et éventuellement un constituant hydrophobe ou partiellement hydrophobe que l'on souhaite incorporer aux bicouches lipidiques.

La solution obtenue est alors atomisée. Cette opération consiste notamment à introduire sous forme de gouttelettes très fines, par exemple au moyen d'un gicleur, la solution dans une enceinte traversée par un fluide gazeux porté à une température supérieure au point d'ébullition du solvant utilisé. Sous l'action de la chaleur, le solvant est vaporisé et entraîné par le courant gazeux. Dans la zone prévue de l'appareil où la température du courant de fluide gazeux devient inférieure à la température de transition du mélange desdits constituants de base une poudre extrêmement fine se forme, cette poudre étant récupérée par un dispositif par exemple, un cyclone et recueillie à la base de ce dispositif. Le mélange ainsi recueilli des constituants de base dissous dans la solution de départ est homogène et constitué de particules extrêmement fines.

Ainsi, le procédé de l'invention permet de traiter des quantités importantes de solution et donc de produire industriellement un mélange pulvérulent qui peut être notamment utilisé pour la production de phases lamellaires lipidiques hydratées tels que des liposomes ou analogues. En effet, comme les constituants de base sont dissous dans un solvant, il n'existe aucun problème d'agitation d'un volume important de solution, et il est possible d'atomiser un débit relativement élevé de solution sans utiliser un appareillage complexe et onéreux. De plus, la puissance énergétique nécessaire à l'atomisation d'une solution et l'évaporation du solvant est nettement inférieure à la puissance énergétique consommée par la technique antérieure de lyophilisation.

Avantageusement, la température d'atomisation est comprise entre 60 °C et 100 °C.

Il est bien entendu possible d'atomiser selon le procédé de l'invention une solution ne contenant qu'un seul constituant, c'est-à-dire un lipide amphiphile, si l'on désire produire des bicouches lipidiques constituées de ce seul constituant. Toutefois, les bicouches lipidiques plus généralement utilisées sont constituées par un mélange de plusieurs constituants, par exemple au moins un constituant lipidique amphiphile, et éventuellement au moins un constituant hydrophobe ou partiellement hydrophobe.

Tout lipide amphiphile susceptible de former des phases lamellaires peut être utilisé dans le procédé de l'invention. Toutefois, il est nécessaire que ces lipides amphiphiles soit seuls, soit en présence de substances partiellement ou totalement hydrophobes, puissent se prêter à l'atomisation, c'est-à-dire que la température de transition du lipide amphiphile ou du mélange soit convenable pour obtenir un atomisat sous forme de poudre.

Dans le cas de la production d'un mélange contenant au moins une substance hydrophobe ou partiellement hydrophobe, pour permettre la formation de phases lipidiques lamellaires, la proportion pondérale de ou des constituants lipidiques amphiphiles dans l'ensemble des constituants de base dissous dans la solution de départ, c'est-à-dire l'ensemble des constituants lipidiques amphiphiles et constituants hydrophobes, doit être supérieure à 50 % au moins, et de préférence supérieure à 70 % environ.

Comme lipide amphiphile convenable, on peut utiliser par exemple des composés appartenant à la famille des phospholipides, des glycolipides ou phospho-aminolipides tels que par exemple une lécithine d'œuf ou de soja, une phosphatidylsérine, un cérébroside ou une sphyngomyéline.

Les substances hydrophobes ou partiellement hydrophobes qui sont convenables pour être mélangées à au moins une substance lipidique amphiphile dans la solution avant atomisation sont extrêmement variées et sont choisies en fonction des propriétés que l'on recherche à conférer d'une part au mélange pulvérulent, et d'autre part, aux phases lamellaires ou aux liposomes produits à partir du mélange pulvérulent.

A titre d'exemple, les substances hydrophobes ou partiellement hydrophobes qui peuvent être utilisées, sont des stérols ou leurs esters, tels que le cholestérol, le β-sitosterol, l'oestradiol, le lanostérol, le stigmastérol, le γ-oryzanol ; des acides gras aliphatiques ou leurs esters tels que par exemple l'acide stéarique, l'acide ximéninique, le myristate d'isopropyle ou analogues ; des acides gras triterpéniques ou leurs esters tels que par exemple l'acide glycyrrhétinique ou analogues ; des alcools gras primaires ou secondaires ou leurs esters tels que par exemple le phosphate dicétylique ; des amines grasses aliphatiques telles que par exemple la stéarylamine ; des acides aminés acylés par un acide gras tels que par exemple l'acide stéaroyl-L-glutamique ; des polypeptides tels que par exemple des polypeptides d'hydrolysat d'élastine ; des vitamines par exemple un tocophérol, avantageusement l'α-tocophérol ; des extraits d'origine animale ou végétale tels que par exemple des extraits embryonnaires, ou des extraits d'aloès ; ou des phénols pouvant comporter un ou plusieurs substituants choisis parmi les groupes radicaux alcoxy, alkyle comprenant de 1 à 4 atomes de carbone, carboxy, formyle, tels que par exemple la vanilline.

Toutefois, cette liste de constituants hydrophobes pouvant être utilisés pour former un mélange pulvérulent selon le procédé de l'invention n'est pas limitative.

De plus, il peut être avantageux mais non indispensable de choisir comme constituant lipidique amphiphile et/ou comme constituant hydrophobe, un composé biologiquement actif et/ou possédant des propriétés organoleptiques et/ou physico-chimiques intéressantes.

4

Par ailleurs, le solvant destiné à préparer la solution devant être atomisée est choisi d'une part selon sa capacité de dissolution des constituants de base décrits ci-dessus, et d'autre part en fonction de son point d'ébullition qui ne doit pas être trop élevé afin d'éviter l'utilisation d'une température d'atomisation qui risquerait de dégrader lesdits constituants de base. Il est également possible, selon l'invention, d'utiliser un mélange de solvants miscibles entre eux dans des proportions appropriées pour répondre aux conditions énoncées ci-dessus. La dissolution des constituants est effectuée avant ou après le mélange des solvants, cette dissolution pouvant demander éventuellement un chauffage léger, par exemple à une température d'environ 40 °C.

Les solvants couramment employés sont notamment, le chloroforme, le méthanol, l'éthanol, le dichlorométhane, ou un mélange chloroforme-méthanol, chloroforme-éthanol à 33 % de méthanol ou d'éthanol respectivement.

Selon une autre caractéristique de l'invention, le fluide gazeux utilisé dans l'atomisation doit être sensiblement et chimiquement inerte vis-à-vis des constituants de base dissous dans la solution de départ. Toutefois, comme l'opération d'atomisation est relativement brève, le temps de contact entre les constituants de base et le gaz vecteur est très court, il est possible d'utiliser généralement de l'air sans risque d'oxydation desdits constituants. Bien entendu, il peut être nécessaire de choisir des gaz plus inertes que l'air, par exemple de l'azote ou analogue notamment dans le cas où la température d'atomisation est relativement élevée, ou l'un des constituants de base est très sensible à l'oxydation.

Avantageusement, le débit gazeux est réglé de façon à éviter qu'une partie de la poudre formée ne soit perdue par entraînement dans le courant de fluide gazeux.

Comme déjà décrit précédemment, le fluide gazeux doit être, à l'entrée de l'enceinte de l'appareil d'atomisation, à une température, appelée température d'atomisation, supérieure à celle d'ébullition du solvant utilisé. Toutefois, la température de ce fluide gazeux ne doit pas empêcher la formation de la poudre dans la zone prévue de l'appareil, c'est-à-dire que dans cette zone, le fluide gazeux doit être à une température inférieure à la température de transition du mélange de constituants dissous dans la solution de départ.

Suivant une variante du procédé selon l'invention, on ajoute à la solution lipidique, avant atomisation, de fines particules solides, de préférence micro-alvéolées, par exemple du Kieselguhr, en quantité au moins égale en poids à celle de l'ensemble des lipides contenus dans la solution et de préférence au moins 3 fois supérieure à ladite quantité de lipide.

Bien entendu, on ajoutera, s'il le faut, une nouvelle quantité de solvant afin que la suspension finale puisse être vaporisable dans l'appareil d'atomisation. De plus, les dimensions desdites particules solides ne devront pas être trop importantes pour ne pas risquer d'obturer le gicleur de l'appareil.

Après atomisation, le complexe lipidique se trouve être intimement mêlé aux dites particules solides sous forme de poudre.

Un avantage de la présente variante du procédé est que l'on peut obtenir une poudre par atomisation même en utilisant des lipides amphiphiles dont la température de fusion est inférieure à 50 °C.

Bien entendu, les procédés de production d'un mélange pulvérulent selon l'invention peuvent être réalisés de façon continue ou discontinue, la poudre étant recueillie directement à la sortie du cyclone soit pour être stockée comme produit, soit pour être utilisée immédiatement, notamment pour la formation de phases lamellaires hydratées tels que des liposomes.

L'invention a également pour objet un mélange pulvérulent de constituants lipidiques avec éventuellement au moins un constituant hydrophobe ou partiellement hydrophobe obtenu par le procédé décrit ci-dessus. Ce mélange présente notamment la caractéristique de pouvoir être très facilement dispersé dans une solution aqueuse.

L'invention a encore pour objet un procédé de préparation de phases lamellaires lipidiques hydratées telles que des liposomes, ou analogues, caractérisé en ce qu'il consiste à disperser le mélange pulvérulent décrit ci-dessus dans un milieu aqueux convenable.

Le mélange pulvérulent obtenu par atomisation est dispersé dans le milieu aqueux par une méthode classique telle que par exemple, le brassage, l'homogénéisation ou la sonication.

Toutefois, le mélange pulvérulent obtenu suivant la variante du procédé d'atomisation de l'invention, dispersé au moyen d'une simple agitation, par exemple à l'aide d'un barreau magnétique, permet d'obtenir une phase lamellaire phospholipidique très hydratée constituée de particules sous forme de liposomes de dimensions particulièrement réduites, de l'ordre de 100 à 300 nanomètres, sans qu'il soit nécessaire d'utiliser les techniques habituelles d'homogénéisation, comme l'ultra-sonication par exemple.

Les particules solides micro-alvéolées contenues dans ladite dispersion peuvent être facilement éliminées par simple filtration, par exemple sur filtre poreux de 10 $\mu$m, qui peut être suivie, si nécessaire, d'une filtration stérilisante.

Avantageusement, le mélange pulvérulent précité peut être recueilli directement dans le récipient où s'effectue l'opération de dispersion dans le milieu aqueux précité, après le moyen de récupération de la poudre, par exemple après le cyclone, à sa partie inférieure. Ainsi, on réalise une production en continu soit de phases lamellaires peu hydratées, soit de liposomes.

Les différents milieux aqueux utilisés pour la préparation de phases lamellaires hydratées peuvent être soit de l'eau, soit des solutions de diverses substances dont le choix dépendra des propriétés que

5

l'on désire conférer aux phases lamellaires hydratées. Par exemple, la solution aqueuse destinée à être encapsulée dans les phases lamellaires ou liposomes peut contenir des substances ayant des propriétés biologiques, organoleptiques, physico-chimiques ou physiologiques intéressantes. A titre d'exemple non limitatif, la solution à encapsuler peut contenir des enzymes, des antibiotiques, des polypeptides tels que des polypeptides d'élastine. Le milieu de dispersion peut être également une solution de chlorure de sodium isotonique.

Selon un premier mode de réalisation de l'invention, il est possible de disperser le mélange pulvérulent obtenu par atomisation directement dans une quantité relativement importante du milieu aqueux pour obtenir directement la concentration pondérale désirée en phases lamellaires.

Par exemple on disperse le mélange pulvérulent atomisé dans un milieu aqueux dans lequel est dissoute éventuellement une substance à encapsuler pour obtenir une concentration pondérale en mélange pulvérulent dans la suspension, inférieure à 50 % environ. On obtient ainsi une suspension de phases lamellaires très hydratées dans lesquelles la substance dissoute est encapsulée. On préfère réaliser des suspensions de concentration pondérale en mélange pulvérulent comprise entre 10 % et 25 %.

Ainsi, le rendement d'encapsulation de la substance dissoute dans le milieu aqueux est compris entre 20 % environ et 50 % environ. Ce rendement d'encapsulation est élevé car il est possible pour obtenir des phases lamellaires hydratées, de disperser le mélange pulvérulent atomisé dans une quantité relativement faible de milieux aqueux.

Suivant une variante du procédé de l'invention, on disperse le mélange pulvérulent atomisé dans une quantité relativement faible d'un milieu aqueux contenant avantageusement les substances que l'on souhaite encapsuler, ladite dispersion étant homogénéisée pour assurer un très bon mélange, par exemple à l'aide d'un broyeur à rouleaux. La concentration pondérale en mélange pulvérulent dans le mélange obtenu est par exemple supérieure à 50 % environ. On obtient ainsi une phase lamellaire peu hydratée.

Cette phase lamellaire peut être soit utilisée directement comme produits industriels, et à ce moment-là, la concentration pondérale en mélange pulvérulent est comprise avantageusement entre 60 % et 75 %, soit cette phase lamellaire peu hydratée peut être diluée dans une nouvelle quantité du milieu aqueux déjà utilisé, ou dans un nouveau milieu aqueux de dispersion, pour former une phase lamellaire très hydratée, c'est-à-dire une suspension de liposomes, avantageusement pour obtenir une concentration pondérale en mélange pulvérulent comprise entre 0,1 % et 10 %. Ainsi, dans cette variante, on obtient un rendement d'encapsulation très élevé qui est supérieur à 50 % environ.

Selon un mode préféré de réalisation de l'invention, le milieu aqueux de dispersion précité est iso-osmotique vis-à-vis de la solution aqueuse à encapsuler. Par ailleurs, ce procédé permet d'obtenir des phases lamellaires ou liposomes de taille très petite, par exemple inférieure ou égale à 3 μm ce qui a pu être vérifié par une étude au microscope électronique.

Il est également possible, si on le désire, d'obtenir une homogénéisation plus poussée de la suspension de liposomes, par exemple au moyen d'une sonication prolongée.

La présente invention a encore pour objet des phases lamellaires lipidiques hydratées telles que des liposomes ou analogues préparées selon le procédé décrit ci-dessus. Avantageusement, les phases lamellaires contiennent des substances encapsulées possédant des propriétés utiles, notamment dans les domaines pharmaceutique, cosmétique, alimentaire ou autre.

Enfin, l'invention a également pour objet des compositions pharmaceutiques ou cosmétiques, contenant des phases lamellaires lipidiques préparées selon le procédé ci-dessus, notamment utilisées comme véhicule pour des substances actives.

Selon les procédés de l'invention, il est possible de réaliser un mélange intime des constituants de base des phases lamellaires lipidiques, à savoir un mélange d'un ou de plusieurs composés lipidiques amphiphiles et d'un ou plusieurs composés hydrophobes, sans nécessiter l'utilisation de composés tensio-actifs. En outre, il est possible de produire des phases lamellaires lipidiques hydratées à partir du mélange pulvérulent, atomisé de l'invention, par dispersion de celui-ci dans une quantité de milieu aqueux relativement faible et en conséquence d'obtenir un rendement d'encapsulation des composés dissous dans le milieu aqueux, élevé.

Par ailleurs, ces procédés permettent de produire des phases lamellaires lipidiques hydratées selon une méthode de fabrication continue ou discontinue. Ils peuvent être de plus utilisés très aisément à échelle industrielle, avec un prix de revient de fabrication inférieur à ceux des procédés connus.

Par ailleurs, le mélange pulvérulent obtenu par atomisation peut être facilement dispersé dans un milieu aqueux ce qui permet d'obtenir des phases lamellaires lipidiques peu hydratées ou des phases lamellaires lipidiques très hydratées.

L'invention sera mieux illustrée et d'autres caractéristiques, détails et avantages de celle-ci apparaîtront plus clairement au vu des exemples suivants non limitatifs, et donnés uniquement à titre d'illustration de la présente invention.

Exemple 1

Dans un bécher de 100 ml on introduit 8 g de phosphatidylsérine et 1 g de β-sitostérol que l'on dissout, à l'aide d'une agitation magnétique, dans 50 ml de chloroforme. A la solution obtenue (solution A) on ajoute, en maintenant l'agitation, 0,1 g de stéaroyl-glutamate de sodium en solution dans 25 ml de méthanol (solution B). Le mélange obtenu est vaporisé dans l'enceinte d'un atomiseur alimentée par de l'air dont on aura réglé la température à 75 °C. On recueille dans un récipient placé à la base de l'appareil une très fine poudre blanche. Cette poudre ainsi obtenue est introduite dans un bécher de 500 ml contenant 100 ml d'une solution contenant 2 % d'hyaluronidase et 0,9 % de chlorure de sodium (solution C). L'homogénéisation du mélange est assurée à température ordinaire grâce à une agitation magnétique maintenue pendant une heure. On obtient une dispersion ayant l'aspect d'un gel. Celle-ci, transvasée dans un récipient de 1,5 ml muni d'une agitation à hélice est diluée dans 900 ml de soluté physiologique (solution aqueuse à 0,9 % de chlorure de sodium). On réalise ainsi une suspension opalescente.

L'observation au microscope électronique de cette suspension révèle la présence de liposomes de taille inférieure à 3 μm.

## Exemple 2

Dans un bécher de 100 ml contenant un mélange composé de 50 ml de chloroforme et de 25 ml de méthanol, on introduit 9,9 g de sphingomyéline et 0,1 g de phosphate dicytélique et on agite. La solution obtenue est atomisée comme dans l'exemple précédent à 75 °C. La poudre recueillie à la sortie de l'appareil est intimement mélangée à 100 ml d'une solution aqueuse à 1 % d'alpha-chymotrypsine et 0,9 de chlorure de sodium, à l'aide d'une agitation magnétique pendant une heure suivie d'une sonication pendant 20 minutes. On dilue, comme dans l'exemple précédent, dans 900 ml de soluté physiologique. On obtient une solution bleutée. La taille des liposomes, déterminée par évaluation du mouvement Brownien des particules (appareil du type Nano-Sizer), est légèrement inférieure à 0,1 μm. On détermine le rendement d'encapsulation de l'α-chymotrypsine après filtration sur gel (sépharose CL-4B) de la solution bleutée. Le rendement d'encapsulation de l'α-chymotrypsine par rapport à l'α-chymotrypsine dissoute dans la solution aqueuse de dispersion est de 40%.

## Exemple 3 à 9

On effectue des préparations de liposomes, suivant la méthode décrite à l'exemple 1, avec les constituants indiqués au Tableau I ci-joint.

### Tableau I

| Ex. | | SOLUTION A dans 50 ml de chloroforme | | | SOLUTION B dans 25 ml de méthanol | | température d'atomisation | SOLUTION C Quantité utilisée : 100 ml |
|---|---|---|---|---|---|---|---|---|
| No. | Nature du produit | Poids (g.) | Nature du produit | Poids (g.) | Nature du produit | Poids (g.) | °C | Nature des produits et concentrations |
| 3 | Cérébroside | 8 | Cholestérol | 2 | Néant | — | 75 | Streptomycine 0,1% Na Cl 0,9% |
| 4 | Lécithine de soja | 9 | β-oestradiol | 1 | Néant | — | 75 | Na Cl 0,9% |
| 5 | Lécithine de soja | 9,9 | Néant | — | Vanilline | 0,1 | 60 | Na Cl 0,9% |
| 6 | Lécithine de soja | 9 | γ-oryzanol | 1 | Néant | — | 80 | Na Cl 0,9% |
| 7 | Lécithine de soja | 8 | Cholestérol | 2 | Néant | — | 75 | Extrait d'aloès 10% Na Cl 0,9% |
| 8 | Lécithine de soja | 9,5 | Cholestérol | 0,5 | Néant | — | 75 | Hydrolysat d'élastine 1% extrait embryonnaire 1% ; Na Cl 0,9% |
| 9 | Lécithine de soja | 8,9 | Cholestérol α-tocophérol | 1 0,1 | Néant | — | 75 | Na Cl 0,9% |

## Exemple 10

Dans un bécher de 100 ml contenant 30 ml de dichlorométhane, on introduit 2 g de lécithine de soja et 0,2 g de sitostérol. On agite à l'aide d'un barreau magnétique jusqu'à dissolution complète. On ajoute ensuite 7 g de Kieselguhr et l'on maintient l'agitation pendant 5 minutes afin d'obtenir une suspension homogène.

Cette suspension est vaporisée dans l'enceinte d'un atomiseur alimenté par un courant d'air dont la température est régulée à 60 °C.

On recueille une poudre très légère.

La poudre ainsi obtenue est introduite dans un bécher de 500 ml contenant une solution aqueuse à 1 % en glycoprotéines de lait et 0,9 % de chlorure de sodium. Le mélange est placé sous agitation magnétique pendant 15 minutes. Il est ensuite filtré sur filtre poreux à 10 micromètres. On obtient ainsi un liquide opalescent dont l'observation au microscope électronique révèle l'existence d'une suspension de liposomes dont la taille, mesurée à l'aide d'un appareil du type Nano-Sizer ® se situe aux environs de 220 nanomètres.

Cette suspension de liposomes peut être stérilisée par filtration stérilisante sur filtre Millipore ® à 0,45 micromètre.

## Exemple 11

La préparation d'une poudre atomisée est réalisée suivant la méthode décrite à l'exemple 1 à partir de 15 g de lécithine de soja et 3 g de lanostérol dans 50 ml de chloroforme.

L'atomisation est conduite à 75 °C. On mélange à la spatule la poudre jaune pâle obtenue avec 6 ml d'une solution aqueuse contenant 5 % de Pyrrolidone-carboxylate de sodium, 5 % de lactate de sodium et 0,9 % de chlorure de sodium, puis on homogénéise à l'aide d'un broyeur à rouleaux.

On obtient une phase lamellaire ayant la consistance d'un gel visqueux dont les caractéristiques sont vérifiées par diffraction aux rayons X. Cette phase lamellaire pourra être utilisée sous cette forme, notamment pour la préparation de produits cosmétiques.

Il est également possible de disperser cette phase lamellaire lipidique peu hydratée dans 200 ml d'une solution aqueuse contenant 0,9 % de chlorure de sodium pour obtenir une suspension de phases lamellaires très hydratées ou liposomes. Le rendement d'encapsulation mesuré de Pyrrolidone-carboxylate de sodium est de 60 %.

## Exemple 12

Dans un bécher de 500 ml, on introduit 0,17 g d'hydrolysat d'élastine dissous dans 100 ml d'éthanol à 96 %. On ajoute ensuite tout en agitant 200 ml de chloroforme et 0,34 g de lécithine de soja. Le mélange est atomisé à 80 °C. La poudre blanche obtenue est mise en suspension dans 50 ml de soluté physiologique au moyen d'une agitation magnétique pendant une heure.

L'observation au microscope électronique révèle l'existence de liposomes de taille inférieure au μm. La sonication de cette suspension permet d'obtenir une solution opalescente bleutée contenant des liposomes dont la taille ne dépasse pas 0,2 μm.

## Exemple 13

Dans un bécher de 500 ml on introduit 90 g de lécithine de soja et 10 g de cholestérol. On disperse ce mélange dans 400 ml de chloroforme. La solution ainsi obtenue est atomisée à 75 °C. L'atomisat, qui apparaît comme une poudre blanche, est directement recueilli dans 1 litre de solution de chlorure de sodium à 0,9 %, contenant 1 g de superoxydismutase (S. O. D.), soumis à une vigoureuse agitation magnétique. A la fin de l'atomisation, on poursuit l'agitation pendant une heure à température ambiante. La suspension laiteuse obtenue est ensuite dispersée dans 9 litres de solution de chlorure de sodium à 0,9 %. On agite à l'homogénéisateur à rotor à palettes pendant 15 minutes à température ambiante. L'observation au microscope électronique permet de visualiser des liposomes de taille moyenne de l'ordre du micromètre.

## Exemple 14

Dans un bécher de 100 ml on introduit 5 g de lécithine de soja, 4 g de cholestérol et 1 g de γ-oryzanol. On dissout ce mélange dans 75 ml de chloroforme. La solution ainsi obtenue est atomisée à 75 °C. L'atomisat est recueilli directement dans 100 ml d'une solution de chlorure de sodium à 0,9 % contenant 1 g d'hydrolysat de collagène. On soumet le mélange ainsi obtenu à une homogénéisation puis la suspension obtenue est ensuite dispersée dans 900 ml d'une solution de chlorure de sodium à 0,9 %.

## Exemple 15

Dans un bécher de 100 ml on introduit 4 g de lécithine de soja, 4 g de sphyngomyéline et 2 g de cholestérol dissous dans 75 ml de chloroforme. La solution obtenue est atomisée à 75 °C. L'atomisat est directement recueilli dans un litre d'une solution à 0,5 % de d-glucose. On soumet cette solution à une vigoureuse agitation pour obtenir une homogénéisation.

## Exemple 16

Dans un bécher de 2 litres, on place 180 g de lécithine de soja et 20 g de stigmastérol que l'on dissout, à l'aide d'une agitation magnétique, dans un litre de dichlorométhane ($CH_2Cl_2$).

Le mélange obtenu est vaporisé dans l'enceinte d'un atomiseur alimenté par de l'air dont on aura réglé la température à 60 °C. On recueille dans un récipient placé à la base de l'appareil une très fine poudre blanche. Cette poudre ainsi obtenue est introduite dans un récipient de 50 litres contenant 20 litres d'une solution de chlorure de sodium à 0,9 % et à 1 % de polypeptides d'élastine. L'homogénéisation du mélange est assurée à température ambiante grâce à un rotor à turbine pendant 1 heure.

La taille des particules mesurée au nano-sizer est de l'ordre de 450 nm. La visualisation au microscope électronique de ces particules démontre l'existence de liposomes.

Les exemples 17 à 22 suivants indiquent des compositions utilisées notamment en cosmétique, et réalisées à partir de suspension de phases lamellaires ou de phases lamellaires elles-mêmes obtenues par le procédé de l'invention qui sont mélangées à un excipient compatible avec l'organisme humain, et notamment avec la peau, et permettant une application de cette suspension sur la peau. Ainsi, le constituant actif est incorporé ou encapsulé dans les phases lamellaires produites industriellement par le procédé de l'invention. La suspension précitée ou la phase lamellaire est mélangée à un excipient convenable pour produire par exemple des crèmes, des laits ou baumes pouvant être appliqués notamment sur la peau.

Selon un procédé analogue il peut être également fabriqué des compositions pharmaceutiques, le constituant encapsulé ou incorporé ayant des propriétés intéressantes d'un point de vue biologique ou physiologique. Ces compositions peuvent être sous une forme solide, pâteuse, liquide selon l'excipient utilisé.

## Exemple 17

Préparation d'une crème de soin hydratante de la peau

Composition : Suspension à l'élastine (selon l'exemple 12) 10 g
Excipient émulsionné huile
dans eau 90 g
Utilisation : applications quotidiennes sur la peau.

## Exemple 18

Crème de soin de la peau à propriétés stimulantes

Composition : Suspension à l'oryzanol 10 g
(selon l'exemple 6)
Excipient émulsionné huile dans
eau 90 g
Utilisation : applications quotidiennes sur la peau.

## Exemple 19

Crème de soin de la peau à propriétés protectrices

Composition : Suspension au Tocophérol 10 g
(selon l'exemple 9)
Excipient émulsionné huile dans
eau 90 g
Utilisation : applications le soir pour préparer la peau à l'exposition aux intempéries le lendemain.

## Exemple 20

Lait hydratant pour le corps

Composition : Suspension à l'extrait d'Aloès 10 g
(selon l'exemple 7)
Excipient émulsionné huile dans
eau 90 g
Utilisation : application après le bain ou après soleil.

## Exemple 21

Baume protecteur pour les lèvres

Composition : phase lamellaire au Lanostérol 10 g (selon l'exemple 11)
Excipient gras 90 g
Utilisation : application sur les lèvres pour protéger des intempéries.

Exemple 22

Traitement stimulant anti-rides

Composition : Suspension à l'extrait embryonnaire
(selon l'exemple 8) 30 g
Excipient gélifié 70 g
Utilisation : application sur le visage une fois par semaine.

Selon l'invention, le solvant utilisé pour produire le mélange pulvérulent par atomisation est un solvant non aqueux.

C'est pourquoi les exemples ne mentionnent à ce sujet que des solvants non aqueux, comme par exemple le chloroforme.

## Revendications

1. Procédé de préparation d'un mélange pulvérulent apte à la production de phases lamellaires lipidiques hydratées, telles que par exemple des liposomes, ledit mélange comprenant au moins un constituant lipidique amphiphile et, éventuellement, au moins un constituant à caractère hydrophobe, caractérisé en ce qu'il comprend la mise en solution du ou desdits constituants lipidiques amphiphiles, et éventuellement du ou des desdits constituants à caractère hydrophobe, dans un solvant non aqueux, et l'atomisation de la solution ainsi obtenue dans un courant de fluide gazeux porté à une température, dite température d'atomisation, supérieure à la température d'ébullition du solvant précité, pour produire ledit mélange pulvérulent, lequel solvant est choisi en fonction de son point d'ébullition qui ne doit pas être trop élevé pour éviter l'utilisation d'une température d'atomisation risquant de dégrader le ou lesdits constituants.

2. Procédé selon la revendication 1, caractérisé en ce que, dans la zone de formation de la poudre, la température du courant de fluide gazeux précité est inférieure à la température de transition du mélange précité de constituant lipidique amphiphile, et éventuellement de constituant hydrophobe.

3. Procédé selon la revendication 2, caractérisée en ce que la température d'atomisation est comprise entre 60 °C et 100 °C.

4. Procédé selon l'une des revendications précédentes, caractérisé en ce que la proportion pondérale du ou des constituants lipidiques amphiphiles précités dans l'ensemble des constituants dissous dans le solvant avant atomisation est supérieure à 50 %.

5. Procédé selon la revendication 4, caractérisé en ce que la proportion pondérale précitée en constituant lipidique amphiphile est supérieure à 70 %.

6. Procédé selon l'une des revendications précédentes, caractérisé en ce qu'au moins l'un des constituants lipidiques amphiphiles ou au moins l'un des constituants hydrophobes précités est un composé biologiquement actif et/ou possède des propriétés organoleptiques, et/ou physico-chimiques.

7. Procédé selon l'une des revendications précédentes, caractérisé en ce que les constituants lipidiques amphiphiles précités sont choisis parmi les composés appartenant à la famille des glycolipides, phospholipides ou phospho-amino-lipides.

8. Procédé selon la revendication 7, caractérisé en ce que les constituants lipidiques amphiphiles précités sont choisis parmi le groupe comprenant : la lécithine de soja ou d'œuf, une phosphatidylsérine, un cérébroside ou une sphyngomyéline.

9. Procédé selon l'une des revendications précédentes, caractérisé en ce que les constituants à caractère hydrophobe ou partiellement hydrophobe précités sont choisis parmi le groupe comprenant les stérols ou leurs esters, les acides gras aliphatiques ou leur esters, les acides gras triterpéniques ou leur esters, des alcools gras primaires ou secondaires ou leurs esters, des amines grasses aliphatiques, des acides aminés acylés par un acide gras, des polypeptides, des vitamines, des extraits d'origine animale ou végétale, ou des phénols pouvant comporter un ou plusieurs substituants choisis parmi les groupes alcoxy, alkyle, comprenant 1 à 4 atomes de carbone, carboxy ou formyle.

10. Procédé selon la revendication 9, caractérisé en ce que les constituants hydrophobes précités sont choisis parmi le groupe comprenant le cholestérol, le β-sitostérol, l'œstradiol, le lanostérol, le stigmastérol, le stigmastérol, le γ-oryzanol, l'acide stéarique, l'acide ximéninique, le myristate d'isopropyle, l'acide glycyrrhétinique, le phosphate dicétylique, la stéarylamine, l'acide stéaroyl-L-glutamique, des polypeptides d'hydrolysat d'élastine, un tocophérol, des extraits embryonnaires, des extraits d'aloès ou la vanilline.

11. Procédé selon l'une des revendications précédentes, caractérisé en ce que le solvant précité est constitué d'un solvant ou d'un mélange de solvant dont au moins l'un est un solvant des constituants lipidiques amphiphiles et/ou des constituants hydrophobes précités, ledit solvant ou mélange de solvant

EP 0 087 993 B2

ayant un point d'ébullition inférieur à la température de dégradation desdits constituants amphiphiles ou hydrophobes dissous.

12. Procédé selon la revendication 11, caractérisé en ce que les solvants sont choisis notamment parmi le groupe comprenant le chloroforme, le méthanol, l'éthanol, le dichlorométhane ou des mélanges chloroforme-méthanol, chloroforme-éthanol, de préférence comportant 33 % de méthanol ou d'éthanol.

13. Procédé selon l'une des revendications précédentes, caractérisé en ce que le fluide gazeux utilisé pour atomiser la solution précitée est un gaz sensiblement chimiquement inerte vis-à-vis des constituants dissous dans ladite solution, tel que l'air ou l'azote.

14. Procédé selon l'une des revendications précédentes, caractérisé en ce que l'on ajoute avant atomisation à la solution précitée desdits constituants lipidiques amphiphiles et éventuellement dudit ou desdits constituants hydrophobes, une quantité de particules solides, de préférence micro-alvéolées, telles que du Kieselguhr, au moins égale en poids à celle de l'ensemble des constituants lipidiques contenus dans ladite solution, et de préférence en quantité au moins trois fois supérieure à ladite quantité de constituants lipidiques.

15. Mélange pulvérulent apte à la production de phases lamellaires lipidiques hydratées, telles que par exemple des liposomes, et comprenant au moins un constituant lipidique amphiphile et éventuellement au moins un constituant à caractère hydrophobe, caractérisé en ce qu'il est obtenu par le procédé selon l'une des revendications précédentes.

16. Procédé de préparation de phases lamellaires lipidiques hydratées telles que des liposomes par exemple, caractérisé en ce qu'il consiste à disperser le mélange pulvérulent selon la revendication 15, dans un milieu aqueux convenable.

17. Procédé selon la revendication 16, caractérisé en ce que le mélange pulvérulent est recueilli directement dans le milieu aqueux précité soumis à une agitation pour disperser ledit mélange pulvérulent.

18. Procédé selon la revendication 16 ou 17, caractérisé en ce qu'on disperse le mélange pulvérulent dans une quantité déterminée du milieu aqueux précité pour obtenir une concentration pondérale désirée en mélange pulvérulent.

19. Procédé selon l'une des revendications 16 à 18, caractérisé en ce que le milieu aqueux précité contient au moins une substance biologiquement active et/ou ayant des propriétés organoleptiques, et/ou physico-chimiques.

20. Procédé selon la revendication 18 ou 19, caractérisé en ce que la concentration pondérale précitée en mélange pulvérulent dans la suspension de phases lamellaires lipidiques hydratées est égale au plus à 50 % et de préférence comprise entre 10 % et 25 %.

21. Procédé selon la revendication 20, caractérisé en ce que le rendement d'encapsulation dans les phases lamellaires lipidiques hydratées précitées de la substance précitée dissoute dans le milieu aqueux précité, est compris entre 20 % et 50 %.

22. Procédé selon l'une des revendications 16 à 19, caractérisé en ce qu'on disperse le mélange pulvérulent précité dans une faible quantité de milieu aqueux pour obtenir des phases lamellaires peu hydratées.

23. Procédé selon la revendication 22, caractérisé en ce que la concentration pondérale en mélange pulvérulent dans le milieu aqueux précité est comprise entre 60 % et 75 %.

24. Procédé selon la revendication 22 ou 23, caractérisé en ce que lesdites phases lamellaires peu hydratées sont obtenues par dispersion du mélange pulvérulent précité dans le milieu aqueux précité par broyage, par exemple au moyen d'un broyeur à rouleaux.

25. Procédé selon l'une des revendications 22 à 24, caractérisé en ce que les phases lamellaires peu hydratées précitées sont dispersées dans le milieu aqueux précité ou un autre milieu aqueux convenable, pour obtenir une concentration pondérale en mélange pulvérulent comprise entre 0,1 % et 10 %.

26. Procédé selon la revendication 25, caractérisé en ce que le rendement d'encapsulation dans les phases lamellaires lipidiques hydratées précitées de la substance précitée dissoute dans le milieu aqueux précité de formation des phases lamellaires peu hydratées, est supérieur à 50 %.

27. Phases lamellaires lipidiques hydratées, caractérisées en ce qu'elles sont obtenues par le procédé selon l'une des revendications 16 à 26.

28. Composition pharmaceutique contenant une dose thérapeutiquement efficace d'un constituant actif, caractérisé en ce que ledit constituant actif est incorporé ou encapsulé dans des phases lamellaires lipidiques hydratées selon la revendication 27, lesdites phases lamellaires étant mélangées avec au moins un excipient acceptable par l'organisme.

29. Composition à usage cosmétique comprenant une quantité efficace d'une substance active, caractérisée en ce que ladite substance est incorporée ou encapsulée dans des phases lamellaires lipidiques hydratées selon la revendication 27, lesdites phases lamellaires lipidiques étant mélangées à au moins un excipient convenable pour obtenir un lait, une pommade, une crème ou un baume.

**Claims**

1. Method of preparing a powdery mixture adapted to the production of hydrated lipidic lamellar

11

EP 0 087 993 B2

phases such for instance as liposomes, the said mixture comprising at least one amphiphile lipidic component and possibly at least one component of hydrophobic character, characterized in that it comprises the dissolving of the said amphiphile lipidic component(s) and possibly of the said component(s) of hydrophobic character in a non-aqueous solvent and the atomization of the solution thus obtained in a stream of gaseous fluid brought to a temperature so-called atomization temperature higher than the boiling temperature of the aforesaid solvent to produce the said powdery mixture, which solvent is selected in accordance with its boiling point which should not be too high to avoid the use of an atomization temperature likely to deteriorate the said component(s).

2. Method according to claim 1, characterized in that within the powder formation area the temperature of the aforesaid stream of gaseous fluid is lower than the transition temperature of the aforesaid mixture of amphiphile lipidic component and possibly of hydrophobic component.

3. Method according to claim 2, characterized in that the atomization temperature is lying between 60 °C and 100 °C.

4. Method according to one of the foregoing claims, characterized in that the ponderal proportion of the aforesaid amphiphile lipidic component(s) in the whole of the components dissolved in the solvent before atomization is higher than 50 %.

5. Method according to claim 4, characterized in that the aforesaid ponderal proportion of amphiphile lipidic component is higher than 70 %.

6. Method according to one of the foregoing claims, characterized in that at least one of the aforesaid amphiphile lipidic components or at least one of the aforesaid hydrophobic components is a biologically active composition and/or having organoleptic and/or physical-chemical properties.

7. Method according to one of the foregoing claims, characterized in that the aforesaid amphiphile lipidic components are selected from the compounds belonging to the family of glycolipids, phospholipids or phospho-amino-lipids.

8. Method according to claim 7, characterized in that the aforesaid amphiphile lipidic components are selected from the group comprising : the soya bean or egg lecithin, a phosphatidylserine, a cerebroside or a sphyngomyeline.

9. Method according to one of the foregoing claims, characterized in that the aforesaid components of hydrophobic or partially hydrophobic character are selected from the group comprising the sterols or their esters, the aliphatic fatty acids or their esters, the triterpenic fatty acids or their esters, primary or secondary fatty alcohols or their esters, fatty aliphatic amines, amino-acids acylated by a fatty acid, polypeptides, vitamins, extracts of animal or vegetable origin or phenols which may comprise one or several substituants selected from the groups alcoxy, alkyl, comprising 1 to 4 carbon atoms, carboxy or formyle.

10. Method according to claim 9, characterized in that the aforesaid hydrophobic components are selected from the group comprising the cholesterol, the β-sitosterol, the oestradiol, the lanosterol, the stigmasterol, the γ-oryzanol, the stearic acid, the ximenic acid, the isopropyl myristate, the glycyrrhetinic acid, the dicethyl phosphate, the stearylamine, the stearoyl-L-glutamic acid, polypeptides of elastine hydrolysate, a tocopherol, embryonic extracts, aloe extracts or the vanillin.

11. Method according to one of the foregoing claims, characterized in that the aforesaid solvent consists of a solvent or of a mixture of solvents at least one of which is a solvent for the aforesaid amphiphile lipidic components and/or the hydrophobic components, the said solvent or mixture of solvents having a boiling point lower than the degradation temperature of the said dissolved amphiphile or hydrophobic components.

12. Method according to claim 11, characterized in that the solvents are selected in particular from the group comprising chloroform, methanol, ethanol, dichloromethane or chloroform-methanol, chloroform-ethanol mixtures preferably including 33 % of methanol or ethanol.

13. Method according to one of the foregoing claims, characterized in that the gaseous fluid used to atomize the aforesaid solution is a gas substantially inert chemically with respect to the components dissolved in the said solution, such as air or nitrogen.

14. Method according to one of the foregoing claims, characterized in that one adds ·before atomization to the aforesaid solution of the said amphiphile lipidic components and possibly of the said hydrophobic component(s), an amount of preferably micro-alveolate solid particles such as Kieselguhr at least equal by weight to that of all the lipidic component contained in the said solution and preferably in an amount at least three times higher than the said amount of lipidic components.

15. Powdery mixture adapted to the production of hydrated lipidic lamellar phases such as for instance liposomes and comprising at least one amphiphile lipidic component and possibly at least one component of hydrophobic character, characterized in that it is obtained by the method according to one of the preceding claims.

16. Method of preparing hydrated lipidic lamellar phases such as liposomes for instance, characterized in that it consists in dispersing the powdery mixture according to claim 15 within a suitable aqueous medium.

17. Method according to claim 16, characterized in that the powdery mixture is directly recovered in the aforesaid aqueous medium subjected to a stirring to disperse the said powdery mixture.

18. Method according to claim 16 or 17, characterized in that one disperses the powdery mixture in a

12

determined amount of the aforesaid aqueous medium to obtain a desired weight concentration of powdery mixture.

19. Method according to one of claims 16 to 18, characterized in that the aforesaid aqueous medium contains at least one substance biologically active and/or having organoleptic and/or physical-chemical properties.

20. Method according to claim 18 or 19, characterized in that the aforesaid weight concentration of powdery mixture in the suspension of hydrated lipidic lamellar phases is at most equal to 50 % and preferably comprised between 10 % and 25 %.

21. Method according to claim 20, characterized in that the output yield of encapsulation into the aforesaid hydrated lipidic lamellar phases of the aforesaid substance dissolved in the aforesaid aqueous medium is comprised between 20 % and 50 %.

22. Method according to one of claims 16 to 19, characterized in that one disperses the aforesaid powdery mixture in a small amount of aqueous medium to obtain low-hydrated lamellar phases.

23. Method according to claim 22, characterized in that the weight concentration of powdery mixture in the aforesaid aqueous medium is comprised between 60 % and 75 %.

24. Method according to claim 22 or 23, characterized in that the little hydrated lamellar phases are obtained through dispersion of the aforesaid powdery mixture in the aforesaid aqueous medium through crushing for instance by means of a roll mill.

25. Method according to one of claims 22 to 24, characterized in that the aforesaid low-hydrated lamellar phases are dispersed in the aforesaid aqueous medium or in another suitable aqueous medium to obtain a weight concentration of powdery mixture comprised between 0.1 % and 10 %.

26. Method according to claim 25, characterized in that the output yield of encapsulation into the aforesaid hydrated lipidic lamellar phases of the aforesaid substance dissolved in the aforesaid aqueous medium of formation of the low-hydrated lamellar phases is above 50 %.

27. Hydrated lipidic lamellar phases, characterized in that they are obtained by the method according to one of claims 16 to 26.

28. Pharmaceutical composition containing a therapeutically effective proportion of an active component, characterized in that the said active component is incorporated or encapsulated into the hydrated lipidic lamellar phases according to claim 27, the said lamellar phases being blended with at least one excipient acceptable by the organism.

29. Composition for cosmetic purposes comprising an effective amount of an active substance, characterized in that the said substance is incorporated or encapsulated into hydrated lipidic lamellar phases according to claim 27, the said lipidic lamellar phases being admixed with at least one suitable excipient to obtain a milk, a salve, a cream or a balsam.


## Patentansprüche

1. Verfahren zur Vorbereitung eines zu Erzeugung von hydratisierten lipidischen lamellenförmigen Phasen wie zum Beispiel Liposomen geeigneten pulverförmigen Gemisches, wobei die besagte Mischung weingstens einen lipidischen amphiphilischen Bestandteil und ggf. wenigstens einen Bestandteil mit wasserabstossender Eigenschaft aufweist, dadurch gekennzeichnet, dass es das Auflösen des bzw. der lipidischen amphiphilischen Bestandteils bzw. Bestandteile und ggf. des bzw. der Bestandteils bzw. Bestandteile mit wasserabstossender Eigenschaft in einem nicht wässerigen Lösungsmittel und die Zertäubung der somit gewonnenen Lösung in einem Strom eines gasförmigen Mediums umfasst, welches auf eine höhere Temperatur, sogenannte Zerstäubungstemperatur, als die Siedetemperatur des vorgenannten Lösungsmittels gebracht wird, um das besagte pulverförmige Gemisch zur erzeugen, welches Lösungsmittel in Abhängigkeit seines Siedepunktes gewält wird, welcher nicht zu hoch sein darf, um die Verwendung einer, den bzw. die besagten Bestandteils bzw. Bestandteile der Abbaugefahr aussetzenden Zerstäubungstemperatur zu vermeiden.

2. Verfahren nach Anspruch 1, dadurch gekennzeichnet, dass in dem Pulverbildungsbereich die Temperatur des vorgenannten Stromes des gasförmigen Mediums niedriger als die Umwandlungstemperatur des vorgenannten Gemisches aus einem lipidischen amphiphilischen Bestandteil und ggf. aus einem wasserabstossenden Bestandteil ist.

3. Verfahren nach Anspruch 2, dadurch gekennzeichnet, dass die Zerstäubungstemperatur zwischen 60 °C und 100 °C liegt.

4. Verfahren nach einem der vorangehenden Ansprüche, dadurch gekennzeichnet, dass das Gewichtsverhältnis des bzw. der vorgenannten lipidischen amphiphilischen Bestandteils bzw. Bestandteile in der Gesamtheit der in dem Lösungsmittel vor Zerstäubung aufgelösten Bestandteile höher als 50 % ist.

5. Verfahren nach Anspruch 4, dadurch gekennzeichnet, dass das vorgenannte Gewichtsverhältnis des lipidischen amphiphilischen Bestandteiles höher als 70 % ist.

6. Verfahren nach einem der vorangehenden Ansprüche, dadurch gekennzeichnet, dass wenigstens einer der lipidischen amphiphilischen Bestandteile oder wenigstens einer der vorgenannten wasserabweisenden Bestandteile eine biologisch wirkende Verbindung ist und/oder organoleptische und/oder physikalische-chemische Eigenschaften besitzt.

7. Verfahren nach einem der vorangehenden Ansprüche, dadurch gekennzeichnet, dass die vorgenannten lipidischen amphiphilischen Bestandteile unter den zu der Familie der Glycolipiden, Phospholipiden oder Phospho-Amino-Lipiden gehörenden Verbindungen gewält werden.

8. Verfahren nach Anspruch 7, dadurch gekennzeichnet, dass die vorgenannten lipidischen amphiphilischen Bestandteile in der das Sojabohnen- oder Eierlezithin, ein Phosphatidylserin, ein Cerebrosid oder ein Sphyngomyelin umfassenden Gruppe gewält werden.

9. Verfahren nach einem der vorangehenden Ansprüche, dadurch gekennzeichnet, dass die vorgenannten Bestandteile mit wasserabweisenden oder teilweise wasserabstossenden Eigenschaften in der die Sterole oder deren Ästere, die aliphatischen Fettsäuren oder deren Ästere, die triterpenischen Fettsäuren oder deren Ästere primäre oder sekundäre Fettalkohole oder deren Ästere, aliphatische Fettamine, durch eine Fettsäure azylierte Aminosäuren, Polypeptide, Vitamine, Extrakte tierischer oder pflanzlicher Herkunft, oder Phenole die einen oder mehrere in den Gruppen Alkoxy, Alkyl, die 1 bis 4 Kohlenstoffatome aufweisen, Carboxy und Formyl gewählten Substituenten, umfassenden Gruppe gewählt werden.

10. Verfahren nach Anspruch 9, dadurch gekennzeichnet, dass die vorgennanten wasserabstossenden Bestandteile in der Cholesterin, β-Sitosterol, Oestradiol, Lanosterol, Stigmasterol, γ-Oryzanol, Stearin-säure, Ximeninsäure, Isopropylmyristat, Glycyrrhetinsäure, Dizetylphosphat, Stearylamin, stearol-L-Glutaminsäure, Polypeptide von Elastinhydrolysat, ein Tocopherol, Embryonalextrakte, Aloesextrakte oder Vanillin umfassenden Gruppe gewält werden.

11. Verfahren nach einem der vorangehenden Ansprüche, dadurch gekennzeichnet, dass das vorgenannte Lösungsmittel aus einem Lösungsmittel oder aus einem Gemisch von Lösungsmitteln von welchen wenigstens eines ein Lösungsmittel für die lipidischen amphiphilischen Bestandteile und/oder die vorgenannten wasserabstossenden Bestandteile ist, besteht, wobei das besagte Lösungsmittel oder Gemisch von Lösungsmitteln einen Siedepunkt, der niedriger als die Abbautemperatur der besagten aufgelösten amphiphilischen oder wasserabstossenden Bestandteile ist, aufweist.

12. Verfahren nach Anspruch 11, dadurch gekennzeichnet, dass die Lösungsmittel insbesondere in der Chloroform, Methanol, Ethanol, Dichloromethan oder Chloroform-Methanol, Chloroform-Ethanol Gemische vorzugsweise mit 33 % Methanol oder Ethanol, umfassenden Gruppe gewält werden.

13. Verfahren nach einem der vorangehenden Ansprüche, dadurch gekennzeichnet, dass das zur Zerstäubung der vorgenannten Lösung verwendete gasförmige Medium ein gegen den in der besagten Lösung aufgelösten Bestandteilen im wesentlichen chemisch inertes Gas wie Luft oder Stickstoff ist.

14. Verfahren nach einem der vorangehenden Ansprüche, dadurch gekennzeichnet, dass man vor der Zertäubung der vorgenannten Lösung der besagten lipidischen amphiphilischen Bestandteile und ggf. des bzw. der besagten wasserabstossenden Bestandteils bzw. Bestandteile eine Menge von vorzugsweise microwabenförmigen Feststoffteilchen wie Kieselguhr zusetzt, welche wenigestens im Gewicht derjenigen der Gesamtheit der in der besagten Lösung enthaltenen lipidischen Bestandteile gleich ist und vorzugsweise mengenmässig wenigstens dreimal grösser als die besagte Menge lipidischer Bestandteile ist.

15. Zur Erzeugung von hydratisierten lipidischen lamellaren Phasen wie z. B. Liposomen geeignetes, wenigstens einen lipidischen amphiphilischen Bestandteil und ggf. wenigstens einen Bestandteil mit wasserabstossender Eigenschaft aufweisendes pulverförmiges Gemisch, dadurch gekennzeichnet, dass es durch das Verfahren gemäss einem der vorangehenden Ansprüche erhalten wird.

16. Verfahren zur Vorbereitung von hydratisierten lipidischen lamellenförmigen Phasen wie z. B. Liposomen, dadurch gekennzeichnet, dass es darin besteht, das pulverförmige Gemisch gemäss Anspruch 15 in einem geeigneten wässerigen Medium zu dispergieren.

17. Verfahren nach Anspruch 16, dadurch gekennzeichnet, dass das pulverförmige Gemisch unmittelbar in dem vorgenannten, einer Rührung zum dispergieren des besagten pulverförmigen Gemisches unverworfenen, wässerigen Medium aufgefangen wird.

18. Verfahren nach Anspruch 16 oder 17, dadurch gekennzeichnet, dass man das pulverförmige Gemisch in einer bestimmten Menge des vorgenannten wässerigen Mediums dispergiert, um eine gewünschte Gewichtskonzentration an pulverförmigem Gemisch zu erhalten.

19. Verfahren nach einem der Ansprüche 16 bis 18, dadurch gekennzeichnet, dass das vorgenannte wässerige Medium wenigstens eine biologisch aktive und/oder organoleptische und/oder physikalische-chemische Eigenschaft aufweisende Substanz enthält.

20. Verfahren nach Anspruch 18 oder 19, dadurch gekennzeichnet, dass die vorgenannte Gewichtskonzentration an pulverförmigen Gemisch in der Aufschwemmung von hydratisierten lipidischen lamellenförmigen Phasen höchstens 50 % beträgt und vorzugsweise zwischen 10 % und 25 % liegt.  .

21. Verfahren nach Anspruch 20, dadurch gekennzeichnet, dass der Ertrag der Einkapselung in den vorgenannten hydratisierten lipidischen lamellenförmigen Phasen der in dem vorgenannten wässerigen Medium aufgelösten vorgenannten Substanz zwischen 20 % und 25 % liegt.

22. Verfahren nach einem der Ansprüche 16 bis 19, dadurch gekennzeichnet, dass man das vorgenannte pulverförmige Gemisch in einer schwachen Menge des wässerigen Mediums dispergiert, um wenig hydratisierte lamellenförmige Phasen zu erhalten.

23. Verfahren nach Anspruch 22, dadurch gekennzeichnet, dass die Gewichtskonzentration an pulverförmigem Gemisch in dem vorgenannten wässerigen Medium zwischen 60 % und 75 % liegt.

24. Verfahren nach Anspruch 22 oder 23, dadurch gekennzeichnet, dass die besagten wenig hydratisierten lamellenförmigen Phasen durch Dispergierung des vorgenannten pulverförmigen gemisches in dem vorgenannten wässerigen Medium durch Zerkleinerung z.B. mittels eines Walzenbrechers erhalten werden.

25. Verfahren nach einem der Ansprüche 22 bis 24, dadurch gekennzeichnet, dass die vorgenannten wenig hydratisierten lamellenförmigen Phasen in dem vorgenannten wässerigen Medium oder in einem anderen geeigneten wässerigen Medium dispergiert werden, um eine zwischen 0,1 % und 10 % liegende Gewichtskonzentration an pulverförmigem Gemisch zu erhalten.

26. Verfahren nach Anspruch 25, dadurch gekennzeichnet, dass die Ausbeute der Einkapselung in den vorgenannten hydratisierten lipidischen lamellenförmigen Phasen der in dem vorgenannten, wenig hydratisierte lamellenförmige Phasen bildenden, wässerigen Medium aufgelösten vorgenannten Substanz grösser als 50 % ist.

27. Hydratisierte lipidische lamellenförmige Phasen, dadurch gekennzeichnet, dass sie durch das Verfahren gemäss einem der Ansprüche 16 bis 26 gewonnen werden.

28. Eine therapeutische wirksame Dosis eines aktiven Bestandteiles enthaltende pharmazeutische Zusammensetzung, dadurch gekennzeichnet, dass der besagte aktive Bestandteil in den hydratisierten lipidischen lamellenförmigen Phasen gemäss Anspruch 27 beigesetzt bzw. eingekapselt ist, wobei die besagten lamellenförmigen Phasen mit wenigstens einem durch den Organismus annehmbaren Bindemittel vermischt sind.

29. Eine wirksame Menge eines aktiven Stoffes enthaltende Zusammensetzung für kosmetische Zwecke, dadurch gekennzeichnet, dass der besagte Stoff in den hydratisierten lipidischen lamellenförmigen Phasen gemäss Anspruch 27 beigesetzt bzw. eingekapselt ist, wobei die besagten lipidischen lamellenförmigen Phasen mit wenigstens einem geeigneten Bindemittel vermischt sind, um eine Milch, eine Salbe, eine Kreme oder einen Balsam zu erhalten.